# EUROPÄISCHE PATENTSCHRIFT

(11) **EP 0 506 617 B1**
(45) Veröffentlichungstag und Bekanntmachung des Hinweises auf die Patenterteilung: **21.08.1996**
(21) Anmeldenummer: 92810202.9
(22) Anmeldetag: 19.03.1992
(51) Int. Cl.: C08G 59/12, C09D 167/00, C09D 163/00, C09D 5/03

(54) **Vernetzungsmittel für Pulverlacke auf Basis von Polyestern**
Curing agent for powder coatings based on polyesters
Agent durcisseur pour revêtements sous forme de poudre à base de polyesters

(30) Priorität: 27.03.1991 CH 939/91
(43) Veröffentlichungstag der Anmeldung: 30.09.1992
(73) Patentinhaber: CIBA-GEIGY AG, 4002 Basel (CH)
(72) Erfinder: Cotting, Jacques-Alain, Dr., CH-1729 Bonnefontaine (CH); Gottis, Philippe, Dr., F-68200 Mulhouse (FR)

(56) Entgegenhaltungen:
- EP-A- 0 365 428
- EP-A- 0 383 601

## Beschreibung

Die Erfindung betrifft die Verwendung bestimmter Polyglycidylverbindungen als Vernetzungsmittel für Pulverlacke auf Basis von Polyesterharzen, die mit Epoxidgruppen reagieren, Pulverlacke mit diesen Komponenten und eine spezielle Verwendung dieser Pulverlacke.

Polyglycidylverbindungen wurden vielfach als Vernetzungsmittel bzw. Härter für Pulverlacke auf Basis von Polyesterharzen vorgeschlagen. Für Außenanstriche, die eine hohe Wetterfestigkeit aufweisen müssen, hat sich in der Praxis besonders Triglycidylisocyanurat als Härter durchgesetzt (s. Ullmann's Encyclopedia of Industrial Chemistry, 5^{th} Ed., Vol A9, p. 559). Triglycidylisocyanurat hat sich dabei zwar bewährt, es ermöglicht allerdings mit Polyesterharzen, mit denen hohe Wetterfestigkeiten erreicht werden, oftmals nur relativ wenig flexible und daher mechanisch anfälligere Anstriche. Werden umgekehrt aber Polyesterharze, die flexiblere Filme bilden, zusammen mit Triglycidylisocyanurat als Härter verwendet, könnte die Wetterbeständigkeit der Anstriche vielfach noch besser sein. Man ist daher derzeit gezwungen, einer der genannten Eigenschaften den Vorrang zu geben und je nach Wichtigkeit der Eigenschaften einen geeigneten Polyesterharz auszuwählen.

In der EP-A-0 383 601 wird die Verwendung von Trimellitsäuretriglycidylester als Härter für Pulverlacke auf Polyesterbasis vorgeschlagen. Trimellitsäuretriglycidylester weist jedoch ebenfalls die oben geschilderten Nachteile von Triglycidylisocyanurat auf.

Die Aufgabe der vorliegenden Erfindung ist nun, Verbindungen zur Verwendung als Härter für Polyesterharzpulverlacke vorzuschlagen, die die Herstellung von Anstrichen erlauben, die gleichzeitig den Ansprüchen an Wetterfestigkeit und Flexibilität in hohem Masse genügen. Die Härter sollen dabei diesen Vorteil insbesondere auch in Verbindung mit Polyestern zeigen, die für triglycidylisocyanurathärtbare Pulverlacke entwickelt wurden.

Diese Aufgabe wird erfindungsgemäß gelöst durch die Verwendung von Polyglycidylverbindungen als Vernetzungsmittel in Pulverlacken auf Basis von Polyesterharzen, die mit Epoxidgruppen reagieren, dadurch gekennzeichnet, daß es sich bei den Verbindungen um Polyglycidylester der Formeln (I) oder (II) handelt: 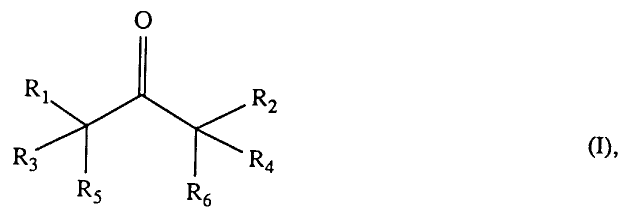 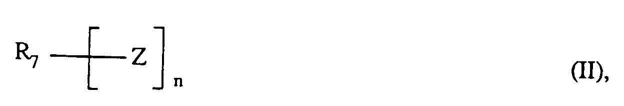 wobei in Formel (I)
R₁,R₂,R₃ und R₄ unabhängig voneinander Wasserstoff, C₁-C₄-Alkyl oder Reste der Formel (III) bedeuten: 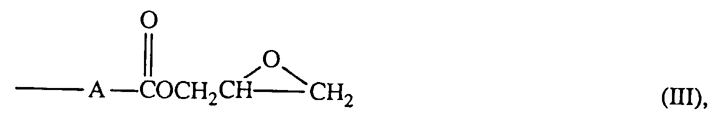 worin A für eine Polymethylengruppe mit 2 bis 4 Kohlenstoffatomen steht, und
R₅ und R₆ unabhängig voneinander Wasserstoff, C₁-C₄-Alkyl, Reste der Formel (III) oder zusammen eine unsubstituierte oder eine mit C₁-C₄-Alkyl substituierte Methylen- oder Polymethylengruppe mit 2 bis 7 Kohlenstoffatomen bedeuten,
jedoch mindestens zwei der Reste R₁ bis R₆ Reste der Formel (III) sind,
und in Formel (II)
n eine ganze Zahl von 2 bis 6,
R₇ einen n-wertigen organischen Rest mit 2 bis 30 Kohlenstoffatomen, und
Z gleiche oder unterschiedliche Reste der Formel (IV) bedeuten: 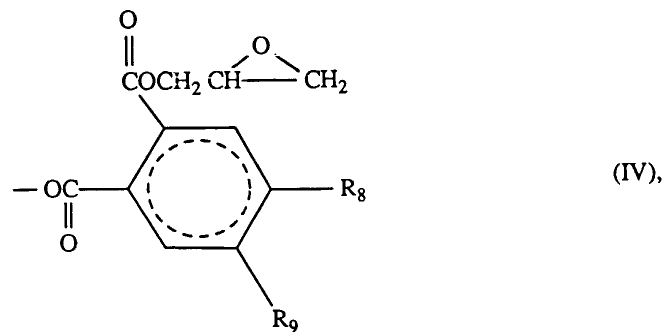 worin R₈ und R₉ entweder unabhängig voneinander Wasserstoff, Chlor, Brom oder C₁-C₄-Alkyl oder eines von beiden einen Rest der Formel (V): 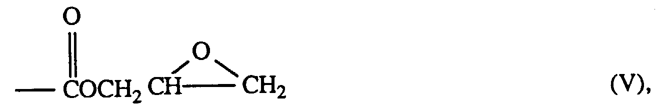 und das andere Wasserstoff, Chlor, Brom oder C₁-C₄-Alkyl bedeuten, und der sechsgliedrige Ring in Formel (IV) aromatisch oder nicht aromatisch ist.

Einen weiteren Gegenstand der Erfindung bilden Pulverlacke, enthaltend zumindest eine Polyglycidylverbindung und einen damit reagierenden Polyesterharz, dadurch gekennzeichnet, daß es sich bei der Verbindung um einen Polyglycidylester der Formeln (I) oder (II) in der oben beschriebenen Bedeutung handelt.

Die Polyglycidylverbindungen der Formel (I) und (II) sind teilweise noch neu.

Verbindungen der Formel (I) sind besonders bevorzugt, wenn in Formel (III) A eine Ethylengruppe bedeutet..

Besonders geeignete Verbindungen der Formel (I) sind beispielsweise der
2,2,5,5-Tetra-(β-carboxyethyl)-cyclopentanontetraglycidylester, der
2,2,6,6-Tetra-(β-carboxyethyl)-cyclohexanontetraglycidylester und der
2,2,4,4-Tetra-(β-carboxyethyl)-pentanon-(3)-tetraglycidylester oder
1,1,3,3-Tetra-(β-carboxyethyl)-acetontetraglycidylester.

Weiterhin bedeuten in der Formel (I) R₁ bis R₄ vorzugsweise alle einen Rest der Formel (III) und R₅ und R₆ vorzugsweise zusammen eine unsubstituierte oder mit C₁-C₄-Alkyl substituierte Methylen- oder Polymethylengruppe mit 2 bis 7 Kohlenstoffatomen, insbesondere eine unsubstituierte Polymethylengruppe mit 2 bis 4 Kohlenstoffatomen. Die Zahl der Alkylsubstituenten kann zwar bis zum Doppelten der Zahl der Kohlenstoffatome der Methylen- oder Polymethylengruppe betragen, sollte aber zweckmässig nur 1 bis 2 betragen.

Die Verbindungen sind aus den entsprechenden Polycarbonsäuren erhältlich, beispielsweise durch Umsetzung der Carbonsäuren mit Epihalogenhydrin zu den Halogenhydrinestern, wobei Halogen vorzugsweise Brom und insbesondere Chlor bedeutet. Die Halogenhydrinester können danach mit halogenwasserstoffbindenden Mitteln zu den entsprechenden Glycidylestern dehydrohalogeniert werden, wie z. B. in der DE-A-2319 815 (= GB 1,409,835) näher beschrieben. Die Herstellung der als Ausgangsprodukte benötigten cycloaliphatischen Polycarbonsäuren kann analog dem britischen Patent Nr. 1,033,697 (= US 3,344,117) erfolgen, die der aliphatischen Polycarbonsäuren z. B. analog der DE-A-26 09 659 (= US 4,102,701).

In Formel (II) bedeutet R₇ vorzugsweise einen zwei- bis sechswertigen, insbesondere einen zwei-, drei- oder vierwertigen, aliphatischen Rest mit 2 bis 10 Kohlenstoffatomen, einen entsprechenden cycloaliphatischen oder aromatischen Rest mit 5 bis 10 Ringkohlenstoffatomen oder einen araliphatischen Rest mit 5 bis 20 Ringkohlenstoffatomen, bei dem gegebenfalls auch einer oder mehrere der aromatischen Kerne bis zur teilweisen oder gänzlichen Sättigung hydriert sein können. Weiterhin können die genannten Reste auch Heteroatome aufweisen. Die Reste R₇ stellen dabei die Rümpfe von Polyalkoholen bzw. Polyolen dar, von denen Hydroxylgruppen in einer Zahl entfernt wurden, die der Zahl n oder insbesondere einer der oben angegebenen Wertigkeiten entspricht. Besonders bevorzugt sind Reste R₇, die sich von gerad- und verzweigtkettigen aliphatischen Polyolen, ableiten, z. B. von Glycolen, wie Ethylen- oder Propylenglycol, von Glycerin, Trimethylolpropan, Erythrit oder Pentaerythrit. Ein weiteres Beispiel ist Sorbit. Ebenfalls als Polyole bevorzugt sind Bisphenoltypen, z. B. 2,2-Di-(4-Hydroxyphenyl)propan oder Di-(4-Hydroxyphenyl)methan, und ähnliche ganz oder teilweise gesättigte Verbindungen, z. B. 2,2-Di-(4-Hydroxycyclohexyl)propan. Gegebenenfalls können die Polyole auch dimerisiert bzw. präpolymerisiert sein, es kann sich z. B. also um Polyetheralkohole handeln, wie Polyethylenglycole oder Bis-trimethylolpropan. Ein Polymerisationsgrad von 2 bis 6 ist bei den Präpolymeren bevorzugt.

Der Kohlenstoffsechsring in Formel (IV) kann entweder aromatisch oder cycloaliphatisch sein, wobei er in letzterem Fall ganz oder nur teilweise gesättigt sein kann. Gegebenenfalls kann er weitere Substituenten, z. B. Chlor, Brom oder C₁-C₄-Alkyl, aufweisen. Dabei sind, abhängig vom Sättigungsgrad des Rings, bis zu 10 Substituenten am Ring möglich; aus praktischen Gründen ist aber eine Zalil von höchstens 4 zweckmäßig. Ganz besonders bevorzugt weist der Ring jedoch nur die Glycidylestergruppen als Substituenten auf.

Die einzelnen Reste Z in Formel (II) können auch verschieden sein. Sie müssen auch nicht die gleiche Zahl Glycidylesterreste aufweisen.

Die Verbindungen der Formel (II) sind besonders für die Erfindung geeignet, insbesondere wenn R₇ ein zwei- bis vierwertiger Rest ist, der sich von einem aliphatischen Polyalkohol oder Polyetherpolyol mit 2 bis 10 Kohlenstoffatomen durch Entfernung von zwei bis vier Hydroxylgruppen ableitet, oder R₇ ein zwei- bis vierwertiger, insbesondere zweiwertiger Rest der folgenden Molekülstruktur 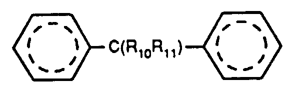 ist, wobei R₁₀ und R₁₁ unabhängig voneinander Wasserstoff oder Methyl bedeuten und die Kohlenstoffsechsringe aromatisch oder nicht aromatisch sein können.

Weiterhin bevorzugt sind die Verbindungen der Formel (II), die insgesamt mindestens 4 Glycidylestergruppen aufweisen.

Die Verbindungen der Formel (II) sind z. B. in folgender Weise zugänglich. Zunächst wird der gewählte Polyalkohol mit Phthalsäure-, Tetrahydrophthalsäure-, Hexahydrophthalsäure- oder Trimellitsäureanhydrid, das gegebenenfalls bis zur teilweisen oder gänzlichen Sättigung hydriert sein kann, oder mit einem Derivat dieser Anhydride, das die oben für R₈und/oder R₉ genannten Substituenten aufweist, im entsprechenden stöchiometrischen Verhältnis zum Halbester umgesetzt. Dabei reagiert im Falle von Trimellitsäureanhydrid, das neben der Anhydridgruppe eine Carboxylgruppe aufweist, praktisch nur die Anhydridgruppe zu einem Halbester mit zwei Carboxylgruppen ab. Die Carboxylgruppen können danach mit Epihalogenhydrin, insbesondere Epichlorhydrin, glycidylisiert werden, wie oben für die Verbindungen der Formel (I) beschrieben.

Die Verbindungen der Formel (I) und (II) sind z. B. als Härter für Pulverlacke auf Basis von Polyesterharzen geeignet, die funktionelle Gruppen aufweisen, die mit Epoxidgruppen reagieren, z. B. Hydroxyl-, Thiol-, Amino-, Amid- oder Carboxylgruppen. Weitere Beispiele geeigneter funktioneller Gruppen sind in Henry Lee, Kris Neville, "Handbook of Epoxy Resins", MacGraw-Hill, Inc. 1967, Appendix 5-1. Bei manchen funktionellen Gruppen kann der Zusatz eines Katalysators zweckmässig sein.

Bevorzugt sind Polyester mit endständigen Carboxylgruppen. Vorzugsweise haben die Polyester eine Säurezahl (angegeben in mg KOH/g Polyester) von 10 bis 100 und ein Molekulargewicht von 500 bis 10000, insbesondere bis 2000. Die verwendeten Polyester sind zweckmäßig bei Raumtemperatur fest und haben eine Glasumwandlungstemperatur von 35 bis 120 °C, vorzugsweise von 40 bis 80 °C.

Polyester, wie im vorigen Abschnitt beschrieben, sind z. B. aus der US-A-3,397,254 bekannt. Sie sind Reaktionsprodukte von Polyolen mit Dicarbonsäuren und gegebenenfalls polyfunktionellen Carbonsäuren oder Carbonsäureanhydriden. Geeignete Polyole sind beispielsweise Ethylenglycol, Propylenglycol, 1,3-Butandiol, 1,4-Butandiol, Neopentandiol, Isopentylglycol, 1,6 Hexandiol, Glycerin, Trimethylolethan, Trimethylolpropan, Erythrit, Pentaerythrit oder Cyclohexandiol. Insbesondere Neopentandiol stellt einen wesentlichen Bestandteil der Polyesterharze dar, die für hochwetterfeste Anstriche geeignet sind. Als Dicarbonsäuren geeignet sind z. B. Isophthalsäure, Terephthalsäure, Phthalsäure, Methylphthalsäuren, Tetrahydrophthalsäure, Methyltetrahydrophthalsäuren, z. B. 4-Methyltetrahydrophthalsäure, Cyclohexandicarbonsäuren, Bernsteinsäure, Glutarsäure, Adipinsäure, Pimelinsäure, Suberinsäure, Azelainsäure, Sebacinsäure, Fumarsäure, Maleinsäure oder 4,4'-Diphenyldicarbonsäure usw. Geeignete Tricarbonsäureanhydride sind z. B. die Anhydride von aliphatischen Tricarbonsäuren, wie 1,2,3-Propantricarbonsäure, von aromatischen Tricarbonsäuren, wie Trimellitsäure (Benzol-1,2,4-tricarbonsäure) und Hemimellitsäure (Benzol-1,2,3-tricarbonsäure) oder von cycloaliphatischen Tricarbonsäuren, wie 6-Methyl-cyclohex-4-en-1,2,3-tricarbonsäure. Geeignete Tetracarbonsäureanhydride sind z. B. Pyromellitsäuredianhydrid oder Benzophenon-3,3',4,4'-tetracarbonsäuredianhydrid.

Die Glycidylverbindungen der Formeln (I) und (II) werden bevorzugt in einer solchen Menge eingesetzt, daß das Verhältnis Carboxylgruppen zu Epoxidgruppen im Pulverlack zwischen 0,5 : 1 und 2 : 1 liegt. Dabei können sie z. B. alleine oder im Gemisch mit anderen Glycidylestern der Formeln (I) oder (II) eingesetzt werden. In manchen Fällen kann es auch vorteilhaft sein, andersartige, für Pulverlacke übliche Epoxidhärter zuzusetzen.

Die Pulverlacke können noch weitere in der Lackindustrie übliche Zusatzstoffe enthalten, wie beispielsweise Lichtschutzmittel, Farbstoffe, Pigmente, z. B. Titandioxidpigment, Entgasungsmittel, z. B. Benzoin, und/oder Verlaufsmittel. Geeignete Verlaufsmittel sind z. B. Polyvinylacetale, wie Polyvinylbutiral, Polyethylenglycol, Polyvinylpyrrolidon, Glycerin, Acrylmischpolymerisate, wie sie z. B. unter den Bezeichnungen Modaflow® [MONSANTO] oder Acrylron® [PROTEX] erhältlich sind.

Eine besondere Ausführungsform der erfindungsgemässen Pulverlacke enthält einen Polyester, der endständige Carboxylgruppen aufweist, und einen Polyglycidylester der Formel (II), wobei R₇ ein zwei- bis vierwertiger Rest ist, der sich von einem aliphatischen Polyalkohol oder Polyetherpolyol mit 2 bis 10 Kohlenstoffatomen durch Entfernung von zwei bis vier Hydroxylgruppen ableitet, oder R₇ ein zwei- bis vierwertiger, insbesondere zweiwertiger Rest mit folgender Molekülstruktur 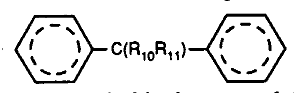 ist, wobei R₁₀ und R₁₁ unabhängig voneinander Wasserstoff oder Methyl bedeuten und die Kohlenstoffsechsringe aromatisch oder nicht aromatisch sein können.

Die erfindungsgemässen Pulverlacke können durch einfaches Mischen der Bestandteile, z. B. in einer Kugelmühle, hergestellt werden. Eine andere Möglichkeit besteht darin, daß man die Bestandteile zusammen aufschmilzt, vermischt und homogenisiert, vorzugsweise in einer Extrusionsmaschine, wie einem Buss-Kokneter, die Masse abkühlt und zerkleinert. Insbesondere dann, wenn Glycidylesterkomponenten flußfähig sind, kann es zweckmäßig sein, auf diese Weise zunächst einen Masterbatch aus den Glycidylesterkomponenten und zumindest einem Teil des Polyestermaterials herzustellen, da sich das Material in dieser Form sauber lagern und bei Gebrauch leicht mit den weiteren gewünschten Komponenten zum endgültigen Pulverlack weiterverarbeiten läßt. Die Pulverlackmischungen weisen vorzugsweise eine Partikelgröße im Bereich von 0,015 bis 500 µm, insbesondere von 10 bis 75 µm, auf.

Die Pulverlacke werden nach Applikation auf den zu beschichtenden Gegenstand bei einer Temperatur von mindestens etwa 100 °C, vorzugsweise 150 bis 250 °C, gehärtet. Für die Härtung sind im allgemeinen etwa 5 bis 60 Minuten erforderlich. Zur Beschichtung geeignet sind alle Materialien, die bei den zur Härtung erforderlichen Temperaturen stabil sind, insbesondere Keramik und Metalle.

Ganz besonders bevorzugt ist die Verwendung der oben beschriebenen Vernetzungsmittel für Polyester, die sich durch einen sehr hohen Anteil aromatischer Dicarbonsäuren, wie Phthalsäure und Terephthalsäure, in Verbindung mit Neopentandiol als alkoholischer Komponente auszeichnen, z. B. zu mindestens 80 Gewichtsprozent, insbesondere zu 90 und mehr Gewichtsprozent, aus Neopentandiol und aromatischen Dicarbonsäuren als Baueinheiten bestehen. Erfindungsgemäße Pulverlacke, die derartige Polyester, die z. B. unter der Bezeichnung Crylcoat®-Typen [UCB], Uralac® [DSM] oder Grilesta® [EMS] im Handel sind, enthalten, sind einerseits sehr witterungsstabil und andererseits zumeist sehr flexibel, sowohl bei plötzlicher als auch bei andauernder mechanischer Belastung.

Wie sich aus den obigen Ausführungen ergibt, sind die erfindungsgemäßen Pulverlacke aufgrund ihrer Witterungsstabilität besonders für Außenanstriche geeignet. Ihre Verwendung zur Herstellung witterungsstabiler Überzüge stellt daher ebenfalls einen Gegenstand der Erfindung dar.

### Beispiel 1: Glycidylierung von 2,2,6,6-Tetra-(β-carboxyethyl)-cyclohexanon

500 g (1,3 Mol) 2,2,6,6-Tetra-(β-carboxyethyl)-cyclohexanon (Herstellung in EP-A-O 366 608 beschrieben) werden mit 3413 g (36,9 Mol) Epichlorhydrin versetzt, und die Temperatur wird auf 100 °C gehalten. Es werden 31 g einer 50%-igen wäßrigen Lösung von Tetramethylammoniumchlorid zugegeben. Die Temperatur wird zwischen 96 und 100 °C gehalten, und der Verlauf der Reaktion wird mit Hilfe einer pH-Elektrode überwacht. Nach etwa 90 min (= Minuten) steigt die Anzeige am pH-Messgerät sprunghaft auf einen Wert von ca. 10 an, was das Ende der Anlagerungsreaktion anzeigt. Nach Entfernung der pH-Elektrode wird das Reaktionsgemisch auf 50 °C gekühlt und es werden nochmals 31 g der 50%-igen wäßrigen Lösung von Tetramethylammoniumchlorid zugegeben. Unter einem Vakuum von 0,09 bis 0,13 bar und bei einer Temperatur von 48 bis 53 °C werden während 250 min 463,7 g wäßrige 50%-ige Natronlauge kontinuierlich zulaufen lassen, wobei das eingebrachte und gebildete Wasser mit Epichlorhydrin als azeotropes Gemisch abdestillieren. Das in einem Wasserabscheider vom Wasser abgetrennte Epichlorhydrin wird kontinuierlich ins Reaktionsgemisch zurückgeführt. Insgesamt können ca. 360 g Wasser abgetrennt werden. Danach wird das Reaktionsgemisch mit 200 ml wäßriger 10%-iger Mononatriumphosphatlösung und dreimal mit 300 ml Wasser gewaschen. Das gewaschene Reaktionsgemisch wird im Rotationsverdampfer unter Wasserstrahlvakuum eingeengt, der Rückstand wird 20 min bei einer Temperatur von 130 °C und einem Druck von 0,0013 bar getrocknet. Man erhält 675,6 g (85% der Theorie) 2,2,6,6-Tetra-(β-carboxyethyl)-cyclohexanontetraglycidylester in Form eines gelben, viskosen Öls (Epoxidgehalt 5,90 Äquivalente, entsprechend 90% der Theorie, Chlorgehalt 1,43%).

### Beispiel 2: Herstellung eines Masterbatches aus 2,2,6,6-Tetra-(β-carboxyethyl)-cyclohexanontetraglycidylester und einem Polyester

In einem Extruder (Kokneter der Firma Buss, Pratteln, CH) wird bei einer Temperatur zwischen 30 und 110 °C, vorzugsweise zwischen 70 und 90 °C, ein Gemisch aus 800 g Crylcoat® 430 (carboxylterminierter Polyester auf Basis von Neopentandiol und Terephthalsäure mit einer Säurezahl von ca. 30 mg KOH/g und einer Glasumwandlungstemperatur (T_{G}) von ca. 70 °C (DSC), hergestellt von UCB, Belgien) und 160 g 2,2,6,6-Tetra-(β-carboxyethyl)-cyclohexanontetraglycidylester homogenisiert. Das abgekühlte Extrudat wird zu Partikeln von ca. 2,4 mm Größe zerkleinert.

Auf diese Weise in eine feste und lagerstabile Form umgewandelt, läßt sich der an sich zähflüßige 2,2,6,6-Tetra-(β-carboxyethyl)-cyclohexanontetraglycidylester auch später besonders leicht zum eigentlichen Pulverlack weiterverarbeiten.

### Beispiel 3: Herstellung eines Pulverlacks

In einem gleichartigen Extruder wie in Beispiel 2 wird bei einer Temperatur von 80 bis 110 °C ein Gemisch aus 420 g des Masterbatches aus Beispiel 2, weiteren 98 g Crylcoat®430, 5,2 g Acrylron®fest (Verlaufsmittel auf Basis eines butylierten Polyacrylats), 1,2 g Benzoin und 259 g Titandioxid homogenisiert. Das abgekühlte Extrudat wird zu einer Partikelgröße von ca. 40 µm zum fertigen Pulverlack vermahlen.

Dieser Pulverlack wird elektrostatisch auf ein Aluminiumblech aufgesprüht. Nach zwanzigminütigem Einbrennen bei einer Temperatur von 200 °C wird ein Lackfilm mit folgenden Eigenschaften erhalten:

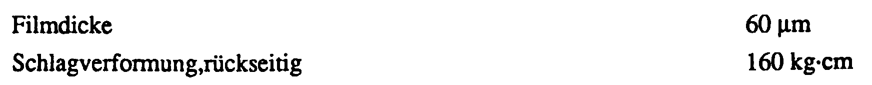

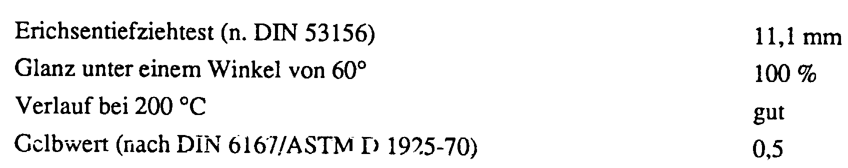

Die Schlagverformung wird bestimmt, indem man einen Stempel bekannten Gewichts aus bestimmter Höhe von hinten auf die beschichtete Fläche fallen läßt. Der angegebene Wert ist das Produkt aus dem Gewicht des Stempels in kg und der größten Höhe in cm, bei der die Beschichtung noch unbeschädigt bleibt.
Der Verlauf wird visuell als mäßig, gut oder sehr gut abgeschätzt.

Beispiel 4: In der gleichen Weise wie in Beispiel 2 wird ein Masterbatch hergestellt, der anstatt Crylcoat® 430 die gleiche Menge Crylcoat® 2988 (carboxylterminierter Polyester aus Neopentandiol und aromatischen Dicarbonsäuren mit einer Säurezahl von ca. 30 Äquivalenten KOH/g und einer Glasumwandlungstemperatur (T_{G}) von ca. 60 °C (DSC), hergestellt von UCB, Belgien) enthält und gemäß Beispiel 3 zu einem Pulverlack verarbeitet. Nach Aufsprühen auf Aluminium und fünfzehnminütigem Einbrennen bei 200 °C wird ein Lackfilm mit folgenden Eigenschaften erhalten:

| | |
|---|---|
| Filmdicke | 55 µm |
| Erichsentiefziehtest (n. DIN 53156) | 6,0 mm |
| Glanz unter einem Winkel von 60° | 90 % |
| Verlauf bei 200 °C | gut |

Die Beschichtung weist bei annähernd gleicher Wetterstabilität eine wesentlich höhere Flexibilität auf als die entsprechenden Beschichtung auf Basis von Triglycidylisocyanurat als Vernetzungsmittel.

### Beispiel 5: Herstellung und Glycidylisierung von Glycerin-1,3-di-(trimellitsäureester)

960,7 g (5 Mol) Trimellitsäureanhydrid werden in 4500 ml Methylisobutylketon bei 115 °C gelöst. Es werden 230,3 g (2,5 Mol) Glycerin während 40 Minuten bei 110 °C zugegeben. Anschließend wird während vier Stunden unter Rückfluß gekocht. Im Verlauf der Reaktion bildet sich eine weiße Suspension des Rohprodukts. Filtration ergibt 464 g weißes Rohprodukt. Dies wird mit 300 ml Methylisobutylketon gereinigt und bei 70 °C unter einem Vakuum von ca. 0,13 bar getrocknet Eine Titration ergibt 11,0 Äquivalente/kg freie Säuregruppen (Theorie: 8,40 Äquivalente).

230 g des getrockneten Produkts werden mit 2350 g (25 Mol) Epichlorhydrin versetzt und die Temperatur wird auf 90 °C gehalten. Es werden 20 g einer 50%-igen wäßerigen Lösung von Tetramethylammoniumchlorid zugegeben, wodurch eine exotherme, jedoch leicht kontrollierbare Reaktion ausgelöst wird. Die Temperatur wird zwischen 88 und 92 °C gehalten, und der Verlauf der Reaktion wird mit Hilfe einer pH-Elektrode überwacht. Nach etwa 90 min steigt die Anzeige am pH-Messgerät sprunghaft auf einen Wert von ca. 10,8 an, was das Ende der Anlagerungsreaktion anzeigt. Nach Entfernung der pH-Elektrode wird das Reaktionsgemisch auf 50 °C gekühlt, und es werden nochmals 20 g der 50%-igen wäßerigen Lösung von Tetramethylammoniumchlorid zugegeben. Unter einem Vakuum von 0,09 bis 0,13 bar und bei einer Innentemperatur von 45 bis 50 °C werden während 300 min 223,5 g wäßerige 50%-ige Natronlauge kontinuierlich zulaufen lassen, wobei das eingebrachte und gebildete Wasser mit Epichlorhydrin als azeotropes Gemisch abdestillieren. Das in einem Wasserabscheider vom Wasser abgetrennte Epichlorhydrin wird kontinuierlich ins Reaktionsgemisch zurückgeführt. Insgesamt können ca. 170 g Wasser abgetrennt werden. Danach wird das Reaktionsgemisch mit 200 ml wäßeriger 10 %-iger Mononatriumphosphatlösung und dreimal mit 300 ml Wasser gewaschen. Das gewaschene Reaktionsgemisch wird im Rotationsverdampfer unter Wasserstrahlvakuum eingeengt, der Rückstand 30 min bei einer Temperatur von 125 °C und einem Druck von 0,0013 bar getrocknet. Man erhält 270 g (61 % der Theorie) Produkt in Form eines gelben, viskosen Öls (Epoxidgehalt 5,2 Äquivalente, entsprechend 91% der Theorie).

### Beispiel 6: Herstellung und Glycidylisierung des Halbesters aus hydriertem Bisphenol A und Phthalsäure

Die Reaktion wird in analoger zu Beispiel 5 durchgeführt, wobei aus 500 g (3,37 Mol) Phthalsäureanhydrid und 405,7 g (1,68 Mol) hydriertem Bisphenol A in 2000 g Methylisobutylketon zunächst 520,9 g des Halbesters als weißer Feststoff mit einem Säuregehalt von 3,67 Äquivalenten/kg (99 % d. Th.) erhalten werden. 520 g (1,91 Äquivalente) davon werden dann unter Verwendung von 3534 g (38,2 Mol) Epichlorhydrin, 168 g wäßeriger 50 %-iger Natronlauge und zweimal 32 g 50 %-iger wäßeriger Tetramethylammoniumchloridlösung umgesetzt und ergeben 607,4 g (98 % d. Th.) des gewünschten gelben und festen Produkts mit einem Epoxidgehalt von 2,97 Äquivalenten/kg (96,4 % d. Th.) und einem Chlorgehalt von 0,51 %.

### Beispiel 7: Herstellung und Glycidylisierung von Glycerin-1,3-diphthalsäureester

Die Reaktion wird in analoger Weise zu Beispiel 5 durchgeführt, wobei aus 888,7 g (6 Mol) Phthalsäureanhydrid und 276,3 g (3 Mol) Glycerin in 960 g Methylisobutylketon zunächst 1165 g (100 % d. Th.) des gewünschten Halbesters als hellgelber Feststoff mit einem Säuregehalt von 5,27 Äquivalenten/kg erhalten werden. 450 g (2,37 Äquivalente) davon werden dann unter Verwendung von 2193 g (23,7 Mol) Epichlorhydrin, 208,6 g wäßriger 50 %-iger Natronlauge und zweimal 21 g 50 %-iger wäßriger Tetramethylammoniumchloridlösung umgesetzt und ergeben 509,4 g (86 % d. Th.) des gewünschten gelben, viskosen Produkts mit einem Epoxidgehalt von 3,38 Äquivalenten/kg (85 % d. Th.) und einem Chlorgehalt von 1,4 %.

### Beispiel 8: Herstellung und Glycidylisierung des Halbesters hydriertem Bisphenol A und Trimellitsäure

Die Reaktion wird in analoger Weise zu Beispiel 5 durchgeführt, wobei aus 300 g (1,56 Mol) Trimellitsäureanhydrid und 187,6 g (0,78 Mol) hydriertem Bisphenol A in 3600 g Methylisobutylketon zunächst 507,5g (100 % d. Th.) des gewünschten Halbesters als hellgelber Feststoff mit einem Säuregehalt von 6,3 Äquivalenten/kg erhalten werden. 505 g (3,18 Äquivalente) davon werden dann unter Verwendung von 5299 g (57,3 Mol) Epichlorhydrin, 280 g wäßriger 50 %-iger Natronlauge und zweimal 46 g 50 %-iger wäßriger Tetramethylammoniumchloridlösung umgesetzt und ergeben 593,0 g (88 % d. Th.) des gewünschten gelben, hoch viskosen Tetraglycidylesters mit einem Epoxidgehalt von 4,32 Äquivalenten/kg (92 % d. Th.) und einem Chlorgehalt von 1,94 %.

### Beispiel 9: Herstellung und Glycidylisierung des Halbesters aus Trimethylolpropan und Hexahydrophthalsäure

330,1 g (2,14 Mol) Hexahydrophthalsäureanhydrid und 93,9 g (0,70 Mol) Trimethylolpropan werden in einem Reaktionsgefäß vorgelegt und unter Rühren auf 130 °C erwärmt. Nach 180 min bei 130 °C ergibt die Titration einer Probe 5,30 Äquivalente/kg freie Säuregruppen (95 % d. Th.). Der gesamte Halbester wird im gleichen Reaktionsgefäß unter Verwendung von 1942 g (21 Mol) Epichlorhydrin, 185 g wäßriger 50 %-iger Natronlauge und zweimal 19 g 50 %-iger wäßriger Tetramethylammoniumchloridlösung wie in Beispiel 5 weiter umgesetzt und ergibt 529,6 g (98,9 % d. Th.) des gewünschten gelben, hoch viskosen Triglycidylesters mit einem Epoxidgehalt von 3,65 Äquivalenten/kg (93,1 % d. Th.) und einem Chlorgehalt von 0,86 %.

### Beispiel 10: Herstellung und Glycidylisierung des Halbesters aus Bis-trimethylolpropan und Hexahydrophthalsäure

539,6 g (3,5 Mol) Hexahydrophthalsäureanhydrid und 219,0 g (0,87 Mol) Bis-trimethylolpropan werden in einem Reaktionsgefäß vorgelegt und unter Rühren auf 130 °C erwärmt. Nach 120 min bei 130 °C ergibt die Titration einer Probe 5,0 Äquivalente/kg freie Säuregruppen. Der gesamte Halbester wird im gleichen Reaktionsgefäß unter Verwendung von 3238 g (35 Mol) Epichlorhydrin, 308 g wäßriger 50 %-iger Natronlauge und zweimal 32 g 50 %-iger wäßriger Tetramethylammoniumchloridlösung wie in Beispiel 5 weiter umgesetzt und ergibt 853,8 g (89 % d. Th.) des gewünschten hellgelben Tetraglycidylesters mit einem Epoxidgehalt von 3,75 Äquivalenten/kg (100 % d. Th.) und einem Chlorgehalt von 1,20 %.

Beispiel 11: In einem Extruder (Kokneter der Fa. BUSS, Pratteln, CH) wird bei einer Temperatur von 90 °C ein Gemisch aus 682 g Crylcoat® 430, 114 g des Glycidylesters von Beispiel 5, 6 g Modaflow® fest, 1,2 g Benzoin und 300 g Titandioxid homogenisiert, wobei vorher ein Masterbatch aus dem zähflüßigen Glycidylester und einer genügenden Menge des Polyesters hergestellt wird, wie in den Beispielen 2 und 3 beschrieben. Das abgekühlte Extrudat wird zu einer Partikelgröße von etwa 40 µm vermahlen. Der erhaltene Pulverlack wird elektrostatisch auf ein Aluminiumblech aufgesprüht und während 30 min bei 200 °C eingebrannt. Es entsteht ein Lackfilm mit folgenden Eigenschaften:

| | |
|---|---|
| Filmdicke | 57 µm |
| Schlagverformung,rückseitig | 160 kg·cm |
| Erichsentiefziehtest (n. DIN 53156) | 9,9 mm |
| Glanz unter einem Winkel von 60° | 90 % |
| Glanz unter einem Winkel von 60° nach 450 h Bewitterung im Weather-O-meter | 65 % |
| Verlauf bei 200 °C | gut |
| Gelbwert (nach DIN 6167/ASTM D 1925-70) | 1,2 |

Die Bewitterungsprüfung fand hier wie auch in den folgenden Beispielen in einem Atlas® Weather-O-Meter unter Verwendung des Bewitterungszyclus mit der Atlas-Benennung NBR 180 statt.

Beispiel 12: Wie in Beispiel 11 beschrieben, wird ein Pulverlack aus 426 g Crylcoat®2988, 75 g des Glycidylesters von Beispiel 5, 5,5 g Modaflow®fest, 1,2 g Benzoin und 266 g Titandioxid homogenisiert. Der erhaltene Pulverlack wird elektrostatisch auf ein Aluminiumblech aufgesprüht und während 30 min bei 200 °C eingebrannt. Es entsteht ein Lackfilm mit folgenden Eigenschaften:

| | |
|---|---|
| Filmdicke | 62 µm |
| Schlagverformung,rückseitig | 160 kg·cm |
| Erichsentiefziehtest (n. DIN 53156) | 9,6 mm |
| Glanz unter einem Winkel von 60° | 90 % |
| Glanz unter einem Winkel von 60° nach 700 h Bewitterung im Weather-O-Meter | 74 % |
| Verlauf bei 200 °C | gut |
| Gelbwert (nach DIN 6167/ASTM D 1925-70) | 1,9 |

Dieser Pulverlack ergibt eine Beschichtung von erheblich höherer Flexibilität, als bei Verwendung einer entsprechenden Formulierung mit Triglycidylisocyanurat als Vernetzungsmittel erhalten wird, und eine viel höhere Bewitterungsbeständigkeit bei gleicher Flexibilität, als man bei Verwendung einer konventionellen Pulverlackzusammensetzung, z. B. auf Basis von Crylcoat®430 als Polyester und Triglycidylisocyanurat als Vernetzungsmittel, erhält.

Beispiel 13: Wie in Beispiel 11 beschrieben, wird ein Pulverlack aus 746 g Crylcoat®430, 190 g des Glycidylesters von Beispiel 8, 10 g Modaflow®fest, 2 g Benzoin und 500 g Titandioxid homogenisiert. Der erhaltene Pulverlack wird elektrostatisch auf ein Aluminiumblech aufgesprüht und während 30 min bei 200 °C eingebrannt. Es entsteht ein Lackfilm mit folgenden Eigenschaften:

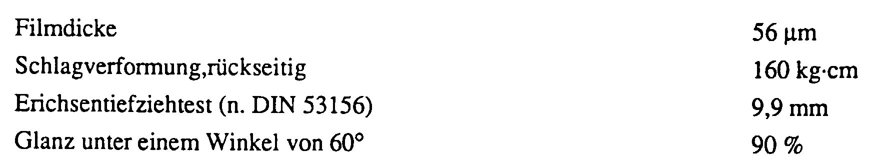

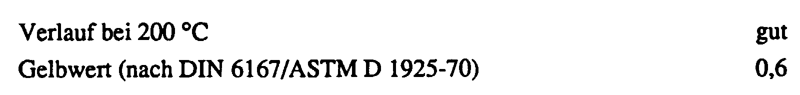

Beispiel 14: Wie in Beispiel 11 beschrieben, wird ein Pulverlack aus 597 g Crylcoat®2988, 160 g des Glycidylesters von Beispiel 8, 8 g Modaflow®fest, 1,6 g Benzoin und 400 g Titandioxid homogenisiert. Der erhaltene Pulverlack wird elektrostatisch auf ein Aluminiumblech aufgesprüht und während 30 min bei 200 °C eingebrannt. Es entsteht ein Lackfilm mit folgenden Eigenschaften:

| | |
|---|---|
| Filmdicke | 61 µm |
| Schlagverformung,rückseitig | 160 kg·cm |
| Erichsentiefziehtest (n. DIN 53156) | 9,8 mm |
| Glanz unter einem Winkel von 60° | 91 % |
| Glanz unter einem Winkel von 60° nach 700 h Bewitterung im Weather-O-Meter | 73 % |
| Verlauf bei 200 °C | gut |
| Gelbwert (nach DIN 6167/ASTM D 1925-70) | 1,4 |

Dieser Pulverlack ergibt eine Beschichtung von erheblich höherer Flexibilität, als bei Verwendung einer entsprechenden Formulierung mit Triglycidylisocyanurat als Vernetzungsmittel erhalten wird, und eine viel höhere Bewitterungsbeständigkeit bei gleicher Flexibilität, als man bei Verwendung einer konventionellen Pulverlackzusammensetzung, z. B. auf Basis von Crylcoat®430 als Polyester und Triglycidylisocyanurat als Vernetzungsmittel, erhält.

Beispiel 15: Wie in Beispiel 11 beschrieben, wird ein Pulverlack aus 630 g Crylcoat®430, 120 g des Glycidylesters von Beispiel 9, 7,5 g Modaflow®fest, 1,5 g Benzoin und 375 g Titandioxid homogenisiert. Der erhaltene Pulverlack wird elektrostatisch auf ein Aluminiumblech aufgesprüht und während 30 min bei 200 °C eingebrannt. Es entsteht ein Lackfilm mit folgenden Eigenschaften:

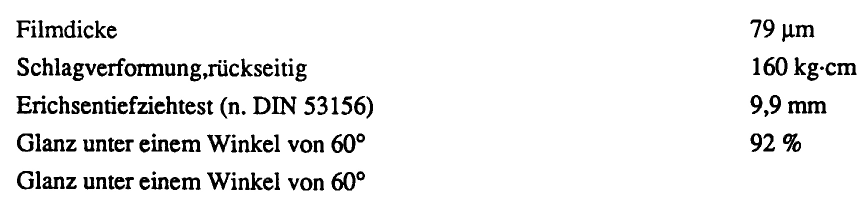

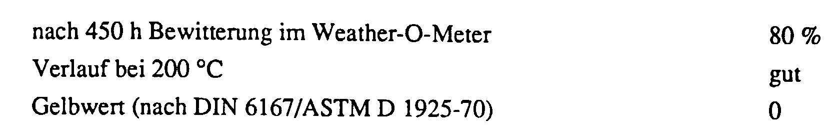

Beispiel 16: Wie in Beispiel 11 beschrieben, wird ein Pulverlack aus 493 g Crylcoat®430, 107 g des Glycidylesters von Beispiel 10, 6,0 g Acrylron®MFP (auf Kieselsäure stabilisiertes butyliertes Polyacrylat als Verlaufsmittel), 1,2 g Benzoin und 300 g Titandioxid homogenisiert. Der erhaltene Pulverlack wird elektrostatisch auf ein Aluminiumblech aufgesprüht und während 30 min bei 200 °C eingebrannt. Es entsteht ein Lackfilm mit folgenden Eigenschaften:

| | |
|---|---|
| Filmdicke | 66 µm |
| Schlagverformung,rückseitig | 160 kg·cm |
| Erichsentiefziehtest (n. DIN 53156) | 10,1 mm |
| Glanz unter einem Winkel von 60° | 87 % |
| Verlauf bei 200 °C | gut |
| Gelbwert (nach DIN 6167/ASTM D 1925-70) | 0 |

## Patentansprüche

1. Verwendung von Polyglycidylverbindungen als Vernetzungsmittel in Pulverlacken auf Basis von Polyesterharzen, die mit Epoxidgruppen reagieren, dadurch gekennzeichnet, daß es sich bei den Verbindungen um Polyglycidylester der Formeln (I) oder (II) handelt: 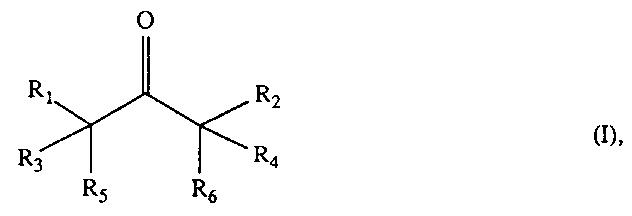 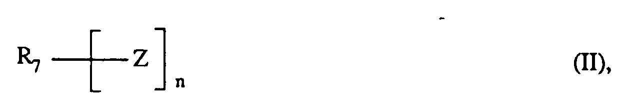 wobei in Formel (I)
R₁,R₂,R₃ und R₄ unabhängig voneinander Wasserstoff, C₁-C₄-Alkyl oder Reste der Formel (III) bedeuten: 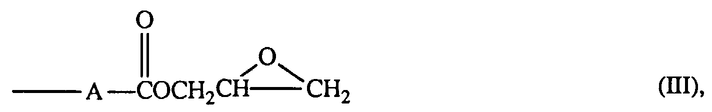 worin A für eine Polymethylengruppe mit 2 bis 4 Kohlenstoffatomen steht, und
R₅ und R₆ unabhängig voneinander Wasserstoff, C₁-C₄-Alkyl, Reste der Formel (III) oder zusammen eine unsubstituierte oder eine mit C₁-C₄-Alkyl substituierte Methylen- oder Polymethylengruppe mit 2 bis 7 Kohlenstoffatomen bedeuten,
jedoch mindestens zwei der Reste R₁ bis R₆ Reste der Formel (III) sind,
und in Formel (II)
n eine ganze Zahl von 2 bis 6,
R₇ einen n-wertigen organischen Rest mit 2 bis 30 Kohlenstoffatomen, und
Z gleiche oder unterschiedliche Reste der Formel (IV) bedeuten: 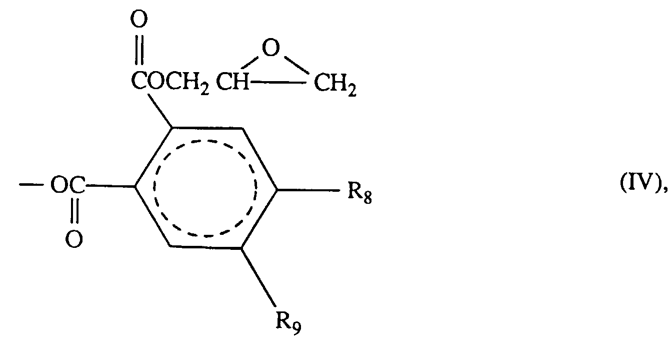 worin R₈ und R₉ entweder unabhängig voneinander Wasserstoff, Chlor, Brom oder C₁-C₄-Alkyl oder eines von beiden einen Rest der Formel (V): 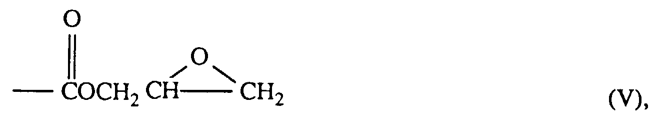 und das andere Wasserstoff, Chlor, Brom oder C₁-C₄-Alkyl bedeuten, und der sechsgliedrige Ring in Formel (IV) aromatisch oder nicht aromatisch ist.

2. Verwendung von Polyglycidylverbindungen nach Anspruch 1, dadurch gekennzeichnet, daß sie die Formel (I) aufweisen und A in Formel (III) eine Ethylengruppe bedeutet.

3. Verwendung von Polyglycidylverbindungen nach einem der Ansprüche 1 oder 2, dadurch gekennzeichnet, daß R₁ bis R₄ alle einen Rest der Formel (III) und R₅ und R₆ zusammen eine Polymethylengruppe mit 2 bis 4 Kohlenstoffatomen bedeuten.

4. Verwendung von Polyglycidylverbindungen nach Anspruch 1, dadurch gekennzeichnet, daß sie die Formel (II) aufweisen und R₇ ein zwei- bis vierwertiger Rest ist, der sich von einem aliphatischen Polyalkohol oder Polyetherpolyol mit 2 bis 10 Kohlenstoffatomen durch Entfernung von zwei bis vier Hydroxylgruppen ableitet, oder R₇ ein zwei- bis vierwertiger Rest mit folgender Molekülstruktur 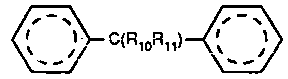 ist, wobei R₁₀ und R₁₁ unabhängig voneinander Wasserstoff oder Methyl bedeuten und die Kohlenstoffsechsringe aromatisch oder nicht aromatisch sein können.

5. Verwendung von Polyglycidylverbindungen der Formel (II)nach Anspruch 1 oder 4, dadurch gekennzeichnet, daß sie vier Glycidylestergruppen aufweisen.

6. Pulverlack, enthaltend zumindest eine Polyglycidylverbindung und einen damit reagierenden Polyesterharz, dadurch gekennzeichnet, daß es sich bei der Verbindung um einen Polyglycidylester der Formel (I) oder (II) handelt: 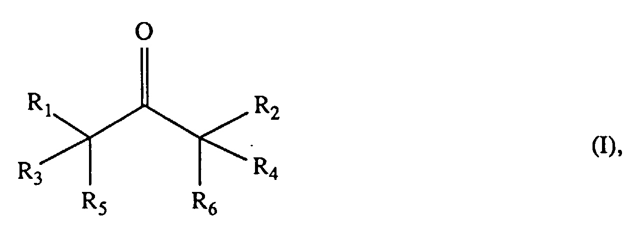 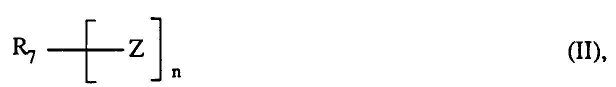 wobei in Formel (I)
R₁,R₂,R₃ und R₄ unabhängig voneinander Wasserstoff, C₁-C₄-Alkyl oder Reste der Formel (III) bedeuten: 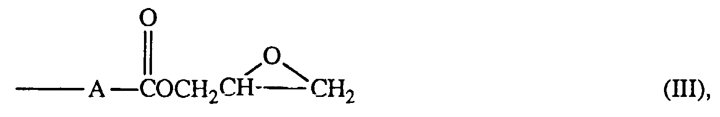 worin A für eine Polymethylengruppe mit 2 bis 4 Kohlenstoffatomen steht, und
R₅ und R₆ unabhängig voneinander Wasserstoff, C₁-C₄-Alkyl, Reste der Formel (Ill) oder zusammen eine unsubstituierte oder eine mit C₁-C₄-Alkyl substituierte Methylen- oder Polymethylengruppe mit 2 bis 7 Kohlenstoffatomen bedeuten,
jedoch mindestens zwei der Reste R₁ bis R₆ Reste der Formel (III) sind,
und in Formel (II)
n eine ganze Zahl von 2 bis 6,
R₇ einen n-wertigen organischen Rest mit 2 bis 30 Kohlenstoffatomen, und
Z gleiche oder unterschiedliche Reste der Formel (IV) bedeuten: 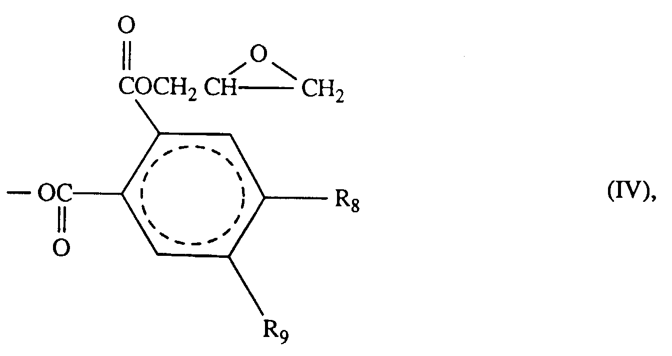 worin R₈ und R₉ entweder unabhängig voneinander Wasserstoff, Chlor, Brom oder C₁-C₄-Alkyl oder eines von beiden einen Rest der Formel (V): 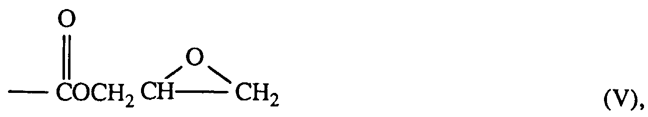 und das andere Wasserstoff, Chlor, Brom oder C₁-C₄-Alkyl bedeuten, und der sechsgliedrige Ring in Formel (IV) aromatisch oder nicht aromatisch ist.

7. Pulverlack nach Anspruch 6, dadurch gekennzeichnet, daß der Polyester endständige Carboxylgruppen aufweist.

8. Pulverlack nach Anspruch 7, dadurch gekennzeichnet, daß er einen Polyglycidylester der Formel (II) enthält, wobei R₇ ein zwei- bis vierwertiger Rest ist, der sich von einem aliphatischen Polyalkohol oder Polyetherpolyol mit 2 bis 10 Kohlenstoffatomen durch Entfernung von zwei bis vier Hydroxylgruppen ableitet, oder R₇ ein zwei- bis vierwertiger Rest mit folgender Molekülstruktur 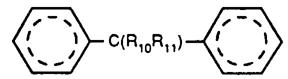 ist, wobei R₁₀ und R₁₁ unabhängig voneinander Wasserstoff oder Methyl bedeuten und die Kohlenstoffsechsringe aromatisch oder nicht aromatisch sein können.

9. Pulverlack nach mindestens einem der Ansprüche 6 bis 8, dadurch gekennzeichnet, daß der Polyester zu 90 Gewichtsprozent und mehr aus Neopentandiol und aromatischen Dicarbonsäuren als Baueinheiten besteht.

10. Verwendung eines Pulverlacks nach einem der Ansprüche 6 bis 9 zur Herstellung von wetterstabilen Überzügen.

## Claims

1. Use of a polyglycidyl compound as crosslinking agent in powder coating compositions based on polyester resins which react with epoxy groups, which compound is a polyglycidyl ester of formula (I) or (II): 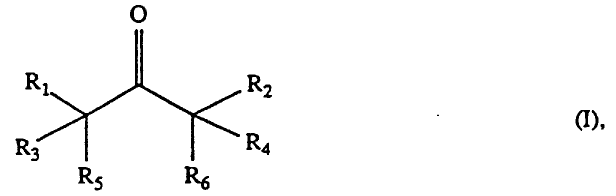 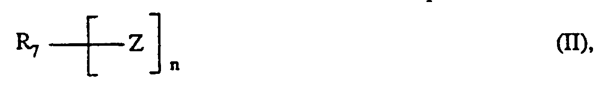 wherein in formula (I)
R₁, R₂, R₃ and R₄ are each independently of one another hydrogen, C₁-C₄alkyl or radicals of formula (III): 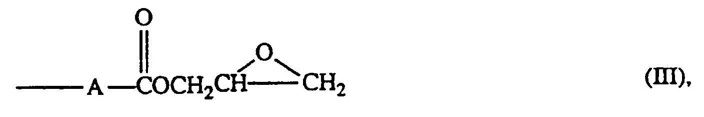 wherein A is a polymethylene group of 2 to 4 carbon atoms, and
R₅ and R₆ are each independently of each other hydrogen, C₁-C₄alkyl or radicals of formula (III) or, together, are an unsubstituted or a C₁-C₄alkyl-substituted methylene or polymethylene group of 2 to 7 carbon atoms, but at least two of the radicals R₁ to R₆ are radicals of formula (III),
and in formula (II)
n is an integer from 2 to 6,
R₇ is an organic radical of valency n of 2 to 30 carbon atoms, and
Z denotes identical or different radicals of formula (IV): 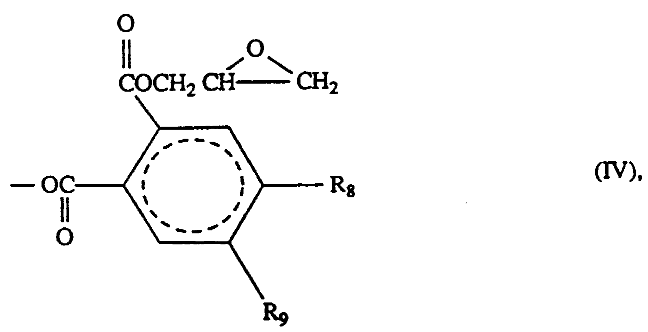
wherein R₈ and R₉ are either each independently of the other hydrogen, chloro, bromo or C₁-C₄alkyl or one is a radical of formula (V): 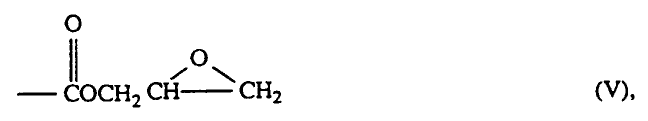 and the other is hydrogen, chloro, bromo or C₁-C₄alkyl, and the six-membered ring in formula(IV) is aromatic or non-aromatic.

2. Use of a polyglycidyl compound of formula (I) according to claim 1, wherein A in formula(III) is an ethylene group.

3. Use of a polyglycidyl compound according to either of claims 1 and 2, wherein R₁ to R₄ are each a radical of formula (III), and R₅ and R₆ together are a polymethylene group of 2 to 4 carbon atoms.

4. Use of a polyglycidyl compound of formula (II) according to claim 1, wherein R₇ is a divalent to tetravalent radical which is derived from an aliphatic polyalcohol or polyetherpolyol of 2 to 10 carbon atoms by removal of two to four hydroxyl groups, or R₇ is a divalent to tetravalent radical of molecular structure 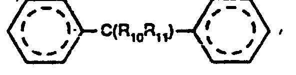 wherein R₁₀ and R₁₁ are each independently of the other hydrogen or methyl and the six-membered carbon rings may be aromatic or non-aromatic.

5. Use of a polyglycidyl compound of formula (II) according to claim 1 or 4, which contains four glycidyl ester groups.

6. A powder coating composition comprising at least one polyglycidyl compound and a polyester resin which reacts therewith, which compound is a polyglycidyl ester of formula (I) or (II): 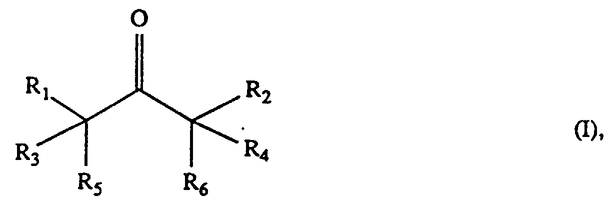 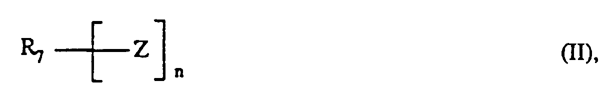 wherein in formula (I)
R₁, R₂, R₃ and R₄ are each independently of one another hydrogen, C₁-C₄alkyl or radicals of formula (III): 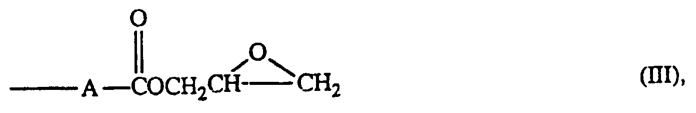 wherein A is a polymethylene group of 2 to 4 carbon atoms, and
R₅ and R₆ are each independently of one another hydrogen, C₁-C₄alkyl or radicals of formula (III) or, together, are an unsubstituted or a C₁-C₄alkyl-substituted methylene or polymethylene group of 2 to 7 carbon atoms, but at least two of the radicals R₁ to R₆ are radicals of formula (III),
and in formula (II)
n is an integer from 2 to 6,
R₇ is an organic radical of valency n of 2 to 30 carbon atoms, and
Z denotes identical or different radicals of formula (IV): 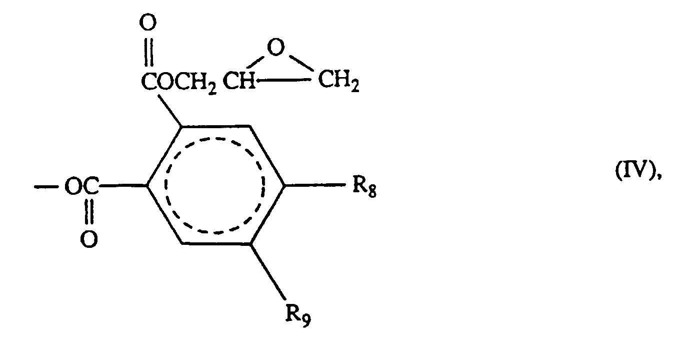 wherein R₈ and R₉ are either each independently of the other hydrogen, chloro, bromo or C₁-C₄alkyl or one is a radical of formula (V): 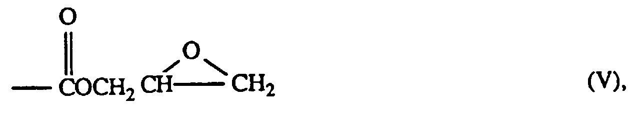 and the other is hydrogen, chloro, bromo or C₁-C₄alkyl, and the six-membered ring in formula (IV) is aromatic or non-aromatic.

7. A powder coating composition according to claim 6, wherein the polyester has terminal carboxyl groups.

8. A powder coating composition according to claim 7, which comprises a polyglycidyl ester of formula (II) wherein R₇ is a divalent to tetravalent radical which is derived from an aliphatic polyalcohol or polyetherpolyol of 2 to 10 carbon atoms by removal of two to four hydroxyl groups, or R₇ is a divalent to tetravalent radical of molecular structure 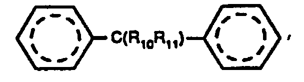 wherein R₁₀ and R₁₁ are each independently of the other hydrogen or methyl and the six-membered carbon rings may be aromatic or non-aromatic.

9. A powder coating composition according to one of claims 6 to 8, wherein the polyester has a content of up to 90 percent and more of neopentanediol and aromatic dicarboxylic acids as structural units.

10. Use of a powder coating composition according to one of claims 6 to 9 for the production of weather resistant coatings.

## Revendications

1. Utilisation des composés polyglycidyliques comme agents réticulants dans les vernis en poudre à base de résines polyesters qui réagissent avec les groupes époxydes, caractérisée en ce que, dans le cas de composés, il s'agit des polyesters polyglycidyliques de formules (I) ou (II) : 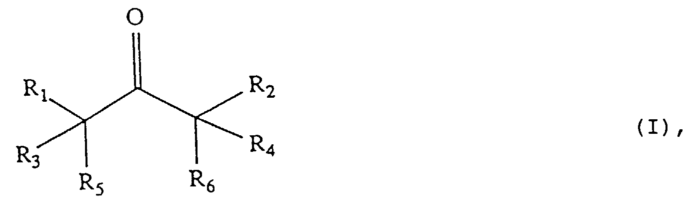 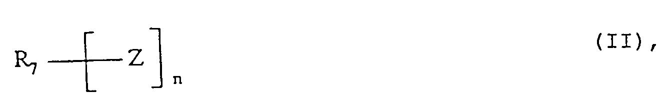 dans la formule (I),
R₁, R₂, R₃ et R₄, indépendamment les uns des autres, représentent l'hydrogène, alkyle en C₁-C₄ ou des radicaux de formule (III) : 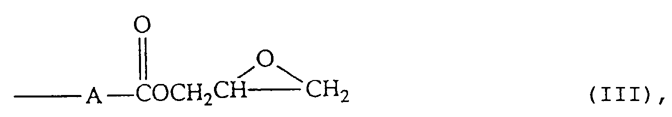 où A représente un groupe polyméthylène avec 2 à 4 atomes de carbone, et
R₅ et R₆, indépendamment l'un de l'autre, représentent l'hydrogène, alkyle en C₁-C₄, des radicaux de formule (III) ou ensemble un groupe méthylène ou polyméthylène avec 2 à 7 atomes de carbone non substitué ou substitué par alkyle en C₁-C₄,
toutefois au moins deux des radicaux R₁ à R₆ sont des radicaux de formule (III),
et dans la formule (II)
n est un nombre entier de 2 à 6,
R₇ représente un radical organique avec n valences avec 2 à 30 atomes de carbone, et
Z représente des radicaux identiques ou différents de formule (IV) : 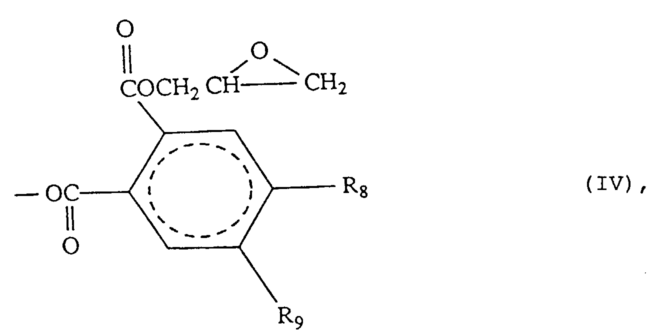 dans laquelle R₈ et R₉, indépendamment l'un de l'autre, sont soit l'hydrogène, le chlore, le brome ou alkyle en C₁-C₄, soit l'un des deux représente un radical formule (V) : 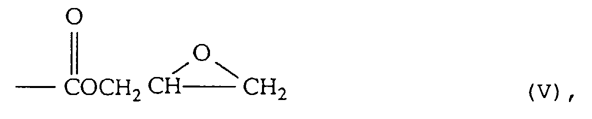 et l'autre représente l'hydrogène, le chlore, le brome ou alkyle en C₁-C₄, et
le cycle à 6 chaînons dans la formule (IV) est aromatique ou non aromatique.

2. Utilisation des composés polyglycidyliques selon la revendication 1, caractérisés en ce qu'ils répondent à la formule (I) et A dans la formule (III) représente un groupe éthylène.

3. Utilisation des composés polyglycidyliques selon l'une des revendications 1 ou 2, caractérisés en ce que tous les R₁ à R₄ représentent un radical de formule (III) et R₅ et R₆ ensemble représentent un groupe polyméthylène avec 2 à 4 atomes de carbone.

4. Utilisation des composés polyglycidyliques selon la revendication 1, caractérisés en ce qu'ils répondent à la formule (II) et R₇ est un radical bi- à tétravalent, qui dérive d'un polyalcool ou polyétherpolyol aliphatique avec 2 à 10 atomes de carbone par élimination de 2 à 4 groupes hydroxyles, ou R₇ représente un radical bi- à tétravalent, plus particulièrement bivalent ayant la structure moléculaire suivante 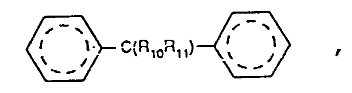 R₁₀ et R₁₁, indépendamment l'un de l'autre, étant l'hydrogène ou méthyle et les cycles carbonés à 6 chaînons pouvant être aromatiques ou non aromatiques.

5. Utilisation des composés polyglycidyliques de formule (II) selon les revendications 1 à 4, caractérisés en ce qu'ils présentent 4 groupes d'esters glycidyliques.

6. Vernis en poudre contenant au moins un composé polyglycidylique et une résine polyester réagissant avec celui-ci, caractérisé en ce que, dans le cas de ces composés, il s'agit d'un ester polyglycidylique de formules (I) ou (II) : 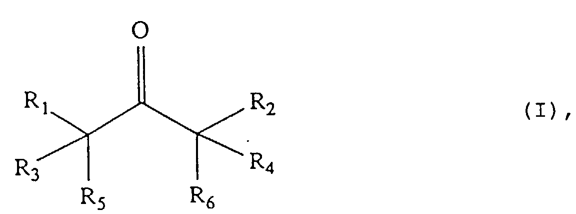 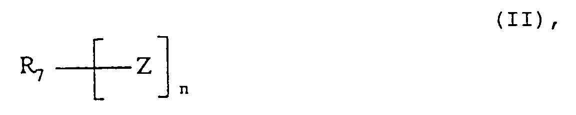 dans la formule (I),
R₁, R₂, R₃ et R₄, indépendamment les uns des autres, représentent l'hydrogène, alkyle en C₁-C₄ ou des radicaux de formule (III) : 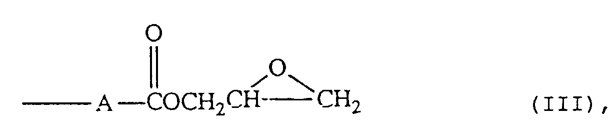 où A représente un groupe polyméthylène avec 2 à 4 atomes de carbone, et
R₅ et R₆, indépendamment l'un de l'autre, représentent l'hydrogène, alkyle en C₁-C₄, des radicaux de formule (III) ou ensemble un groupe méthylène ou polyméthylène avec 2 à 7 atomes de carbone non substitué ou substitué par alkyle en C₁-C₄,
toutefois au moins deux des radicaux R₁ à R₆ sont des radicaux de formule (III),
et dans la formule (II)
n est un nombre entier de 2 à 6,
R₇ représente un radical organique avec n valences avec 2 à 30 atomes de carbone, et
Z représente des radicaux identiques ou différents de formule (IV) : 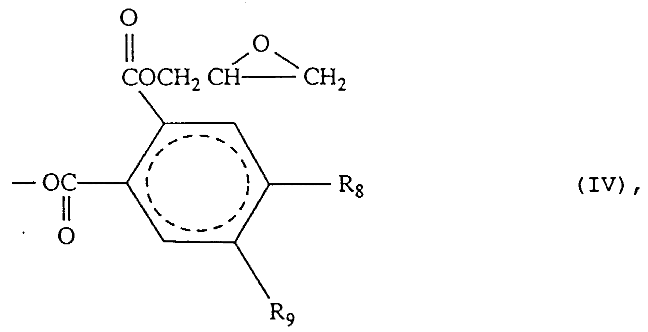 dans laquelle R₈ et R₉, indépendamment l'un de l'autre, sont soit l'hydrogène, le chlore, le brome ou alkyle en C₁-C₄, soit l'un des deux représente un radical formule (V) : 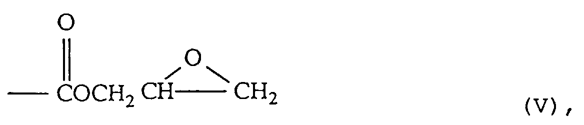 et l'autre représente l'hydrogène, le chlore, le brome ou alkyle en C₁-C₄, et
le cycle à 6 chaînons dans la formule (IV) est aromatique ou non aromatique.

7. Vernis en poudre selon la revendication 6, caractérisé en ce que le polyester présente des groupes carboxyles terminaux.

8. Vernis en poudre selon la revendication 7, caractérisé en ce qu'il contient un ester polyglycidylique de formule (II), R₇ étant un radical bi- à tétravalent, qui dérive d'un polyalcool ou polyétherpolyol aliphatique avec 2 à 10 atomes de carbone par élimination de 2 à 4 groupes hydroxyles, ou R7 représente un radical bi- à tétravalent ayant la structure moléculaire suivante 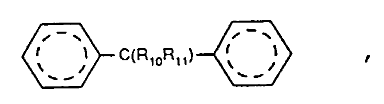 R₁₀ et R₁₁, indépendamment l'un de l'autre, étant l'hydrogène ou méthyle et les cycles carbonés à 6 chaînons pouvant être aromatiques ou non aromatiques.

9. Vernis en poudre selon au moins l'une des revendications 6 à 8, caractérisé en ce que le polyester se compose à 90 % en poids et plus de néopentanediol et des acides dicarboxyliques aromatiques en tant qu'éléments de structure.

10. Utilisation d'un vernis en poudre selon les revendications 6 à 9 pour la préparation de revêtements résistant aux intempéries.
